# EUROPÄISCHE PATENTSCHRIFT

(11) **EP 0 509 273 B1**
(45) Veröffentlichungstag und Bekanntmachung des Hinweises auf die Patenterteilung: **25.01.1995**
(21) Anmeldenummer: 92105030.8
(22) Anmeldetag: 24.03.1992
(51) Int. Cl.: C07D 307/28, C07C 403/24

(54) **2,5-Bis-(1',1'-dialkoxy-2'-propyl)-2,5-dihydrofurane, Verfahren zu deren Herstellung sowie deren Verwendung zur Herstellung von Carotinoiden**
2,5-Bis-(1',1'-dialkoxy-2'-propyl)-2,5-dihydrofurans, process for their preparation and their use in preparing carotenoids
2,5-Bis-(1',1'-dialkoxy-2'-propyl)-2,5-dihydrofurannes, procédé pour leur préparation, et leur utilisation dans la préparation de caroténoîdes

(30) Priorität: 15.04.1991 DE 4112272
(43) Veröffentlichungstag der Anmeldung: 21.10.1992
(73) Patentinhaber: BASF Aktiengesellschaft, 67063 Ludwigshafen (DE)
(72) Erfinder: Frank, Jürgen, Dr., W-6703 Limburgerhof (DE); Rheude, Udo, Dr., W-6900 Heidelberg (DE); Schulz, Bernhard, Dr., W-6830 Schwetzingen (DE); Paust, Joachim, Dr., W-6708 Neuhofen (DE); Hickmann, Eckhard, Dr., W-6701 Dannstadt-Schauernheim (DE)

(56) Entgegenhaltungen:
- CH-A- 321 106
- JOURNAL OF ORGANIC CHEMISTRY. Bd. 47, Nr. 11, 1982, EASTON US Seiten 2130 -2134; M. ROSENBERGER ET AL.: 'Canthaxanthin. A New Total Synthesis'
- SYNTHESIS. Dezember 1987, STUTTGART DE Seiten 1043 - 1054; T. MUKAIYAMA ET AL.:
- 'Cross-Coupling Reactions Based on Acetals'
- ANGEWANDTE CHEMIE. INTERNATIONAL EDITION. Bd. 16, Nr. 7, 1977, WEINHEIM DESeiten 423 - 429; H. POMMER: 'The Wittig Reaction in Industrial Practice'
- CHEMISTRY LETTERS. 1989, TOKYO JP Seiten 1277 - 1280; T. MUKAIYAMA ET AL.: 'TheAddition Reaction of Acetals (Aldehydes) to simple Olefins by the Use of a NewCatalyst System'

## Beschreibung

Die Erfindung betrifft 2,5-Bis-(1',1'-dialkoxy-2'-propyl)-2,5-dihydrofurane der allgemeinen Formel I 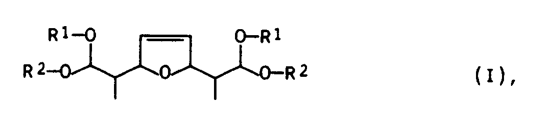
in der R¹ und R² gleich oder verschieden sein können und für eine Alkylgruppe mit 1 bis 4, vorzugsweise 1 oder 2 C-Atomen steht, ein einfaches Verfahren zu deren Herstellung sowie deren Verwendung zur Herstellung von 2,7-Dimethyl-octa-2,4,6-trien-1,8-dial sowie von symmetrischen Carotinoiden.

Ein vorteilhaftes Verfahren zur Herstellung von begehrten symmetrischen Carotinoiden, wie den C₄₀-Carotinoiden der allgemeinen Formel IV β-Carotin, Canthaxanthin und Astaxanthin beruht auf der beidseitigen Verknüpfung von 2,7-Dimethyl-octa-2,4,6-trien-1,8-dial mit den entsprechenden C₁₅-Triphenylphosphoniumsalzen der Formel V in einer Wittig-Reaktion (vgl. DE 28 01 908).

Die Herstellung des dafür benötigten C₁₀-Dialdehydes 2,7-Dimethyl-octa-2,4,6-trien-1,8-dial erfolgt nach dem heutigen Stand der Technik aus Methylglyoxaldimethylacetal und 1,4-Bis-(dimethylphosphono)-but-2-en durch Wittig-Horner-Reaktion und Acetalhydrolyse (vgl. beispielsweise Angew. Chem. 89, (1977), 437-443).

Die Verfahren zur Herstellung der Vorprodukte Methylglyoxalacetal und 1,4-Bis-(dimethylphosphono)-but-2-en sind sicherheitstechnisch recht aufwendig und machen so den C₁₀-Dialdehyd zu einer kostspieligen Synthesekomponente.

So wird Methylglyoxalacetal zweistufig aus Aceton gewonnen, indem man das Aceton zunächst mit Methylnitrit nitrosiert und das Oxim anschließend mit Methanol zum gewünschten Produkt weiterverarbeitet. 1,4-Bis-(dimethylphosphono)-but-2-en gewinnt man aus But-2-en-1,4-diol über das gesundheitlich sehr bedenkliche 1,4-Dibrom-but-2-en und dessen Umsetzung mit Trimethylphosphit. Nebenprodukt ist das ebenfalls gesundheitsgefährdende Brommethan.

Weiterhin ist aus CH 321 106 bekannt, daß man den C₁₀-Dialdehyd auch durch Umsetzungen zwischen Fumardialdehydacetalen oder Maleindialdehydacetalen und Alkylpropenylethern herstellen kann. Die hierbei als Zwischenprodukte auftretenden C₁₀-Dietheracetale werden in saurem Medium zum C₁₀-Dialdehyd hydrolysiert, der kristallin isoliert werden kann.

Nachteilig an diesem Verfahren ist, daß der Fumar- bzw. der Maleindialdehyd durch Partialhydrierung aus acetylenischen Vorstufen hergestellt werden. Dem Fachmann ist bekannt, daß solche Partialhydrierungen schwierig durchzuführen sind und nur unter strikter Einhaltung genau definierter Reaktionsbedingungen mit ausreichender Selektivität gelingen.

Aus der CH 321 106 geht weiterhin hervor, daß die C₁₀-Dietheracetale thermisch empfindliche Stoffe sind, die nicht gereinigt werden können und daher bevorzugt undestilliert zu dem C₁₀-Dialdehyd weiterverarbeitet werden.

Neben dem wirtschaftlichen Nachteil seines hohen Preises besitzt der C₁₀-Dialdehyd den physikalischen Nachteil einer hohen Kristallisationstendenz, die seine Gewinnung zwar erleichtert, in der Folge jedoch seine Umsetzung mit anderen Komponenten erschwert. Seine Verwendung in Carotinoidsynthesen erfordert daher den Einsatz großer Lösungsmittelmengen sowie großer Apparaturen.

Es war daher die Aufgabe der Erfindung, einen vorteilhafteren Herstellungsweg für den C₁₀-Dialdehyd oder ein C₁₀-Dialdehydäquivalent und damit für symmetrische Carotinoide zu finden, der die Nachteile des Standes der Technik überwindet.

Es wurde nun gefunden, daß man durch Bereitstellen der erfindungsgemäßen 2,5-Bis-(1',1'-dialkoxy-2'-propyl)-2,5-dihydrofurane der allgemeinen Formel I, deren vorteilhafte Herstellungsmöglichkeit sowie deren einfache überführbarkeit in den C₁₀-Dialdehyd bzw. in symmetrische Carotinoide die Probleme der bisherigen C₁₀-Dialdehyd-Gewinnung und -Verwendung überraschend einfach lösen kann.

Gegenstand der Erfindung ist daher neben den 2,5-Dihydrofuranen der Formel I ein Verfahren zu deren Herstellung, das dadurch gekennzeichnet ist, daß man ein 2,5-Dialkoxy-2,5-dihydrofuran der allgemeinen Formel II 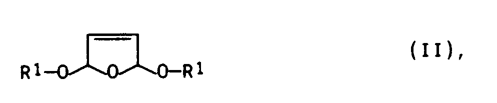
in der R¹ für eine Alkylgruppe mit 1 bis 4 C-Atomen, vorzugsweise eine Methylgruppe oder eine Ethylgruppe steht, in Gegenwart eines sauren Katalysators und gegebenenfalls in Gegenwart eines Lösungsmittels bei Temperaturen von -78°C bis zum Siedepunkt des Reaktionsgemisches mit einem Alkylpropenylether der Formel III 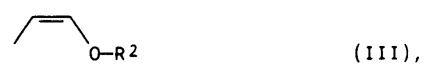
in der R² für eine Alkylgruppe mit 1 bis 4 C-Atomen, vorzugsweise eine Methylgruppe oder Ethylgruppe steht, umsetzt.

Die als Ausgangsstoffe benötigten 2,5-Dialkoxy-2,5-dihydrofurane der Formel II sind als Zwischenprodukte zur Herstellung des bioziden Succinaldehyds industriell in größerem Umfang verfügbar. Man erhält sie beispielsweise durch elektrochemische Oxidation von Furan in Alkanolen.

Als saure Katalysatoren kommen für das erfindungsgemäße Verfahren Protonensäuren oder Lewissäuren in Betracht. Mit besonderem Vorteil arbeitet man in Gegenwart von Lewis-Säuren.

Als Protonensäuren kommen insbesondere Halogen, Phosphor oder Schwefel enthaltende Säuren in Betracht. Genannt seien beispielsweise HCl, HBr, HClO₄, H₃PO₄, H₂SO₄, CH₃SO₃H oder p-Toluolsulfonsäure.

Unter dem Ausdruck Lewissäure verstehen wir erfindungsgemäß ein Halogenid eines Elements der 2., 3. oder 4. Hauptgruppe, bzw. der 2., 4. oder 8. Nebengruppe des Periodensystems der Elemente. Beispielsweise seien MgCl₂, MgBr₂, BF₃, AlCl₃, SnCl₄, ZnCl₂, ZnBr₂, TiCl₄, FeCl₃, CoCl₂ und CoBr₂ genannt. Man kann auch durch Kombination zweier für sich alleine nicht katalytisch aktiver Stoffe die Reaktion katalysieren. Systeme dieser Art sind vor kurzem in der Literatur bekannt geworden (Chem. Lett. 1989, 1277-1280).

Man kann die Umsetzung entweder lösungsmittelfrei oder in Gegenwart eines Lösungsmittels durchführen. Mit Vorteil arbeitet man in Gegenwart eines Lösungsmittels. Als Lösungsmittel sind diejenigen Stoffe geeignet, die sowohl den Katalysator als auch die Reaktionskomponenten lösen und die unter den Reaktionsbedingungen stabil sind. Vorzugsweise verwendet man Ester einer C₁-C₄-Carbonsäure mit einem C₁-C₄-Alkanol, insbesondere Essigsäureethylester. Doch auch C₃-C₆-Ether, wie Diethylether, 1,2-Dimethoxyethan, 1,2-Diethoxyethan oder cyclische Ether, wie Tetrahydrofuran oder 1,4-Dioxan und C₂-C₇-Nitrile, wie Acetonitril, Propionitril oder Benzonitril sowie C₃-C₈-Ketone, wie Aceton, Methylethylketon, Diethylketon, Methylisobutylketon, Cyclohexanon oder Acetophenon können recht gut als Lösungsmittel verwendet werden. Mit Vorteil verwendet man einen Carbonsäureester, wie Essigsäureethylester als Lösungsmittel.

Die Wahl des Lösungsmittels folgt aus Zweckmäßigkeitsüberlegungen und hat keinen prinzipiellen Einfluß auf das Gelingen der Reaktion.

Die Reaktion führt man im allgemeinen bei Temperaturen von -78°C bis zum Siedepunkt des verwendeten Lösungsmittels, vorzugsweise bei -30 bis +30°C, insbesondere bei -15 bis +15°C durch.

In der wahl des Dialkoxydihydrofurans bzw. des Alkylpropenylethers ist man nicht auf die Verwendung ganz bestimmter Derivate angewiesen, sondern kann beliebige Komponenten einsetzen. Die in der Formel I eingesetzten Reste R¹ und R² können gleich oder verschieden sein, je nachdem, ob die Alkoxygruppe des Dihydrofurans gleich oder ungleich derjenigen des Propenylethers ist. Setzt man Ausgangsmaterialien mit unterschiedlichen Alkoxygruppen miteinander um, z. B. 2,5-Dimethoxydihydrofuran und Ethylpropenylether, so gewinnt man als Reaktionsprodukt die statistische Mischung der Acetale der Formel I mit Methoxy- und Ethoxygruppen.

Zur Durchführung des erfindungsgemäßen Verfahrens legt man zweckmäßigerweise den Katalysator in dem Lösungsmittel vor und gibt die Reaktionskomponenten der Formel II und III bei der geeignetsten Reaktionstemperatur zu.

Die erfindungsgemäßen Verbindungen der Formel I sind unzersetzt destillierbare Flüssigkeiten.

Zur Herstellung von dem C₁₀-Dialdehyd 2,7-Dimethyl-octa-2,4,6-trien-1,8-dial kann man das erfindungsgemäß erhaltene Isomerengemisch in Gegenwart von Wasser mit Säure behandeln. Den C₁₀-Dialdehyd kann man dann entweder isolieren oder vorzugsweise ohne Isolierung direkt mit dem gewünschten Triphenylphosphoniumsalz behandeln und so eine Wittig-Olefinierung zu einem symmetrischen Carotinoid durchführen. Ganz besonders vorteilhaft ist dabei, daß in vielen Fällen zur Hydrolyse der Bisacetale der allgemeinen Formel I sowie zur Spaltung des Dihydrofuranringes der Verbindungen der Formel I die Säurewirkung der wäßrigen Lösung eines Alkyl- oder Alkylidentriphenylphosphoniumhydrogensulfats ausreicht. Nach erfolgter Acetalspaltung führt man dann durch Zugabe einer geeigneten Base die Wittig-Reaktion durch. So erhält man aus den Dihydrofuranen der Formel I und beispielsweise beta-Ionylidenethyltriphenylphosphoniumhydrogensulfat in einem Eintopfverfahren das begehrte beta-Carotin. Bei Verwendung von weniger sauren Triphenylphosphoniumsalzen ist unter Umständen eine an die Wittig-Reaktion anschließende Säurebehandlung zwecks Spaltung des Dihydrofuranringes im Reaktionsprodukt erforderlich.

Gegenstand der Erfindung ist daher auch die Verwendung der 2,5-Bis-(1',1'-dialkoxy-2'-propyl)-2,5-dihydrofurane der Formel I zur Herstellung von 2,7-Dimethyl-octa-2,4,6-trien-1,8-dial sowie zur Herstellung von symmetrischen C₄₀-Carotinoiden der allgemeinen Formel IV 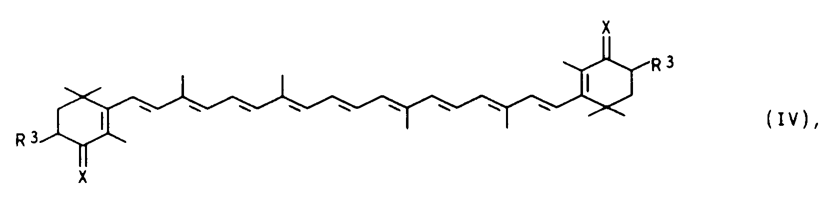
in der R³ für ein Wasserstoffatom oder die OH-Gruppe steht und X für zwei Wasserstoffatome oder ein Sauerstoffatom steht, und ein Verfahren zur Herstellung von C₄₀-Carotinoiden der allgemeinen Formel IV 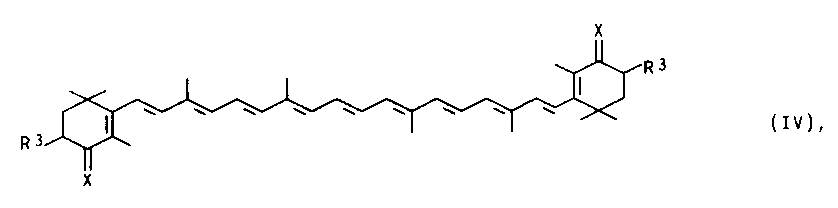
in der R³ für ein Wasserstoffatom oder die OH-Gruppe steht und X für zwei Wasserstoffatome oder ein Sauerstoffatom steht, das dadurch gekennzeichnet ist, daß man ein 2,5-Bis-(1',1'-dialkoxy-2'-propyl)-2,5-dihydrofuran der allgemeinen Formel I zunächst mit einem C₁₅-Triphenylphosphoniumsalz der allgemeinen Formel V 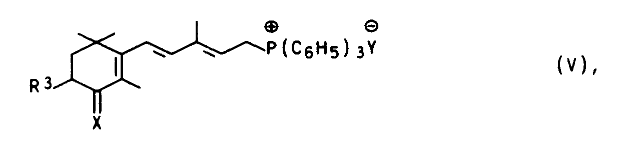
in der R³ und X die oben angegebene Bedeutung haben und Y für ein Äquivalent eines geeigneten Säurerestes, insbesondere für HSO₄^{⊖} oder Cl^{⊖} steht, versetzt, und das hierbei durch Hydrolyse aus dem Bisacetal der Formel I gebildete Dial durch Zufügen einer geeigneten starken Base in einer Wittig-Reaktion mit dem aus dem Triphenylphosphoniumsalz der Formel V gebildeten Carbeniumion und gegebenenfalls anschließende Säurebehandlung zur Spaltung des gegebenenfalls in dem Reaktionsprodukt noch enthaltenen Dihydrofuranringes zu dem gewünschten C₄₀-Carotinoid umsetzt.

### Beispiel 1

In 100 ml Essigsäureethylester wurden 0,5 g FeCl₃ bei -10°C vorgelegt und hierzu eine Mischung aus 79 g (0,5 mol) 2,5-Diethoxy-2,5-dihydrofuran und 86 g (1 mol) Ethylpropenylether so zugetropft, daß die Temperatur zwischen -8 und -10°C blieb. Nach dem Zutropfen wurden 3 ml Triethanolamin zur Zerstörung des sauren Katalysators zugegeben, der erhaltene Niederschlag wurde abfiltriert und das Filtrat destilliert. Man erhielt 93 g 2,5-Bis-(1',1'-diethyloxy-2-propyl)-2,5-dihydrofuran (Isomerengemisch) mit einem Siedebereich von 120 bis 180°C/1 mbar. Das entspricht einer Ausbeute von 56 % der Theorie.

### Beispiel 2

Zu einer Lösung von 1 g ZnCl₂ in 50 ml Essigsäureethylester wurden bei 50°C zunächst 32,5 g (0,25 mol) 2,5-Dimethoxy-2,5-dihydrofuran zugegeben. Anschließend tropfte man 43 g (0,5 mol) Ethylpropenylether bei 50°C zu. Nach 2stündigem Nachrühren bei 50°C wurden in der Reaktionsmischung 21,2 % 2,5-Bis-(1'-methoxy-1'-ethoxy-2'-propyl)-2,5-dihydrofuran (statistische Mischung) gefunden. Das bedeutet bei einer Menge an Reaktionsgemisch von 126 g eine Ausbeute von 36 % der Theorie.

### Beispiel 3

Zu einer Lösung von 1 g SnCl₄ und 0,4 g Benzonitril in 25 ml Essigsäureethylester gab man bei -10°C 32,5 g (0,25 mol) 2,5-Dimethoxy-2,5-dihydrofuran hinzu und tropfte anschließend bei Temperaturen zwischen -8 und -10°C innerhalb von 15 Minuten (min) 43 g (0,5 mol) Ethylpropenylether zu. Nach 20 minütigem Nachrühren bei -10°C wurden in der Reaktionsmasse 31 % 2,5-Bis-(1'-methoxy-1'-ethoxy-2'-propyl)-2,5-dihydrofuran gefunden. Das bedeutet bei einer Menge an Reaktionsmasse von 100 g eine Ausbeute von 42 % der Theorie.

### Beispiel 4

### Synthese von β-Carotin

Eine gemäß EP 140 hergestellte Suspension, enthaltend 0,2 mol β-Jonylidenethyl-triphenylphosphoniumhydrogensulfat wurde mit 33 g (ca. 0,1 mol) des gemäß Beispiel 2 hergestellten 2,5-Bis-(1'-methoxy-1'-ethoxy-2'-propyl)-2,5-dihydrofurans versetzt und das so erhaltene Reaktionsgemisch 6 Stunden (h) bei 80°C gerührt. Anschließend kühlte man auf Raumtemperatur (RT) ab und tropfte bei RT in 30 min 50 ml einer 25 %igen wäßrigen Ammoniaklösung zu. Nach 2 h wurden 300 ml Heptan zugefügt, auf 60°C erwärmt und anschließend ca. 500 ml 80 %iges Methanol so zugefügt, daß man eine klare Unterphase erhielt. Die Salze und Triphenylphosphinoxid enthaltende Unterphase wurde abgetrennt. Die das β-Carotin gelöst oder suspendiert enthaltende Oberphase wurde dreimal mit 400 ml eines 80 %igen Methanols gewaschen und anschließend soviel Heptan abdestilliert, daß eine gut rührbare Maische von β-Carotin in Heptan zurückblieb. Diese Maische wurde 24 h unter Rückfluß zum Sieden erhitzt um das erhaltene β-Carotin in die all-trans-Form zu isomerisieren. Nach Abkühlen auf RT und Verdünnen mit Heptan wurde abgesaugt. Man erhielt 19 g kristallines, spezifikationsgerechtes β-Carotin. Die Ausbeute an all-trans-β-Carotin betrug 35 %, bezogen auf das β-Jonyliden-ethyl-triphenylphosphoniumsalz. In der Mutterlauge verblieben noch 9 g eines cis/trans-Isomerengemisches von β-Carotin. Die Gesamtausbeute betrug somit 52 % der Theorie. Das Verfahren wurde noch nicht optimiert.

## Patentansprüche

1. 2,5-Bis-(1',1'-dialkoxy-2'-propyl)-2,5-dihydrofurane der allgemeinen Formel I 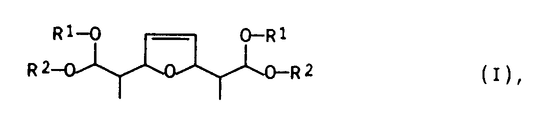 in der R¹ und R² gleich oder verschieden sein können und für eine Alkylgruppe mit 1 bis 4 C-Atomen stehen können.

2. Verfahren zur Herstellung der 2,5-Bis-(1',1'-dialkoxy-2'-propyl)-2,5-dihydrofurane der allgemeinen Formel I gemäß Anspruch 1, dadurch gekennzeichnet, daß man ein 2,5-Dialkoxy-2,5-dihydrofuran der allgemeinen Formel II 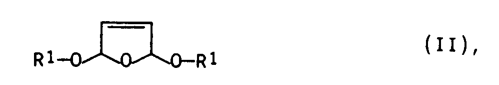 in der R¹ für eine Alkylgruppe mit 1 bis 4 C-Atomen steht, in Gegenwart eines sauren Katalysators und gegebenenfalls eines Lösungsmittels bei Temperaturen von -78°C bis zum Siedepunkt des Reaktionsgemisches mit einem Alkylpropenylether der Formel III 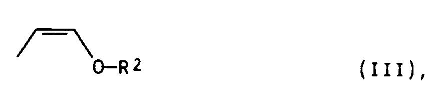 in der R² für eine Alkylgruppe mit 1 bis 4 C-Atomen steht, umsetzt.

3. Verfahren gemäß Anspruch 2, dadurch gekennzeichnet, daß man als sauren Katalysator eine Protonensäure oder eine Lewissäure verwendet.

4. Verfahren gemäß Anspruch 3, dadurch gekennzeichnet, daß man als Lewissäure ein Halogenid eines Elementes der 2., 3. oder 4. Hauptgruppe oder der 2., 4. oder 8. Nebengruppe des Periodensystems der Elemente verwendet.

5. Verfahren gemäß Anspruch 4, dadurch gekennzeichnet, daß man als Lewissäure MgCl₂, MgBr₂, BF₃, AlCl₃, SnCl₄, ZnCl₂, ZnBr₂, TiCl₄, FeCl₃, CoCl₂ oder CoBr₂ verwendet.

6. Verfahren gemäß Anspruch 2, dadurch gekennzeichnet, daß man die Umsetzung bei Temperaturen von -30 bis +30°C durchführt.

7. Verfahren gemäß Anspruch 2, dadurch gekennzeichnet, daß man die Umsetzung in Gegenwart von einem Keton mit 3 bis 8 C-Atomen, einem Nitril mit 2 bis 7 C-Atomen, einem aliphatischen oder cycloaliphatischen Ether mit 3 bis 6 C-Atomen oder einem Ester aus einer aliphatischen Carbonsäure mit 1 bis 4 C-Atomen und einem Alkohol mit 1 bis 4 C-Atomen als Lösungsmittel durchführt.

8. Verwendung der 2,5-Bis-(1',1'-dialkoxy-2'-propyl)-2,5-dihydrofurane der allgemeinen Formel I gemäß Anspruch 1 zur Herstellung von 2,7-Dimethyl-octa-2,4,6-trien-1,8-dial.

9. Verwendung der 2,5-Bis-(1',1'-dialkoxy-2'-propyl)-2,5-dihydrofurane der allgemeinen Formel I gemäß Anspruch 1 zur Herstellung von symmetrischen C₄₀-Carotinoiden der allgemeinen Formel IV 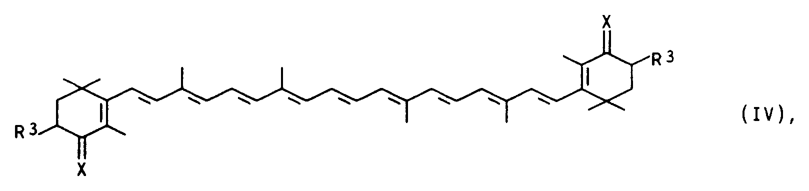 in der R³ für ein Wasserstoffatom oder die OH-Gruppe steht und X für zwei Wasserstoffatome oder ein Sauerstoffatom steht.

10. Verfahren zur Herstellung von symmetrischen C₄₀-Carotinoiden der allgemeinen Formel IV 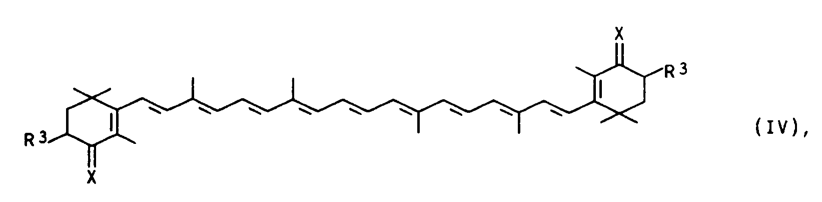 in der R³ für ein Wasserstoffatom oder die OH-Gruppe steht und X für zwei Wasserstoffatome oder ein Sauerstoffatom steht, dadurch gekennzeichnet, daß man ein 2,5-Bis-(1',1'-dialkoxy-2'-propyl)-2,5-dihydrofuran der allgemeinen Formel I 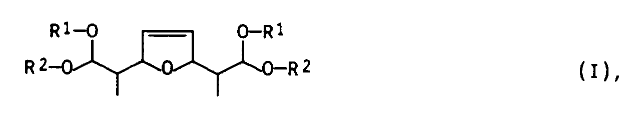 in der R¹ und R² die in Anspruch 1 angegebene Bedeutung hat, zunächst mit einem C₁₅-Triphenylphosphoniumsalz der allgemeinen Formel V 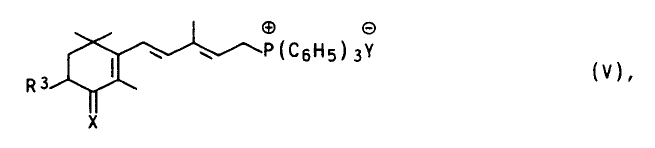 in der
R³ für ein Wasserstoffatom oder eine OH-Gruppe steht,
X für zwei Wasserstoffatome oder ein Sauerstoffatom steht, und
Y für ein Äquivalent eines geeigneten Säurerestes steht,
versetzt und das hierbei durch Hydrolyse aus dem Bisacetal der Formel I gebildete Dial durch Zufügen einer geeigneten starken Base in einer Wittig-Reaktion mit dem aus dem Phosphoniumsalz der Formel V gebildeten Carbeniumion und gegebenenfalls anschließende Säurebehandlung zu dem C₄₀-Carotinoid umsetzt.

## Claims

1. A 2,5-bis(1,1-dialkoxy-2-propyl)-2,5-dihydrofuran of the formula I 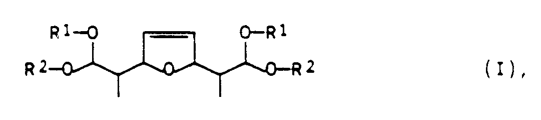 where R¹ and R² can be identical or different and can be alkyl of 1 to 4 carbons.

2. A process for preparing a 2,5-bis(1,1-dialkoxy-2-propyl)-2,5-dihydrofuran of the formula I as claimed in claim 1, which comprises reacting a 2,5-dialkoxy-2,5-dihydrofuran of the formula II 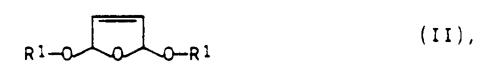 where R¹ is alkyl of 1 to 4 carbons, in the presence of an acid catalyst and in the presence or absence of a solvent, at from -78°C to the boiling point of the reaction mixture, with an alkyl propenyl ether of the formula III 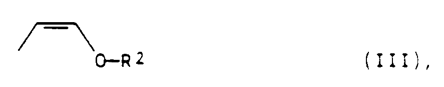 where R² is alkyl of 1 to 4 carbons.

3. A process as claimed in claim 2, wherein a protic acid or a Lewis acid is used as acid catalyst.

4. A process as claimed in claim 3, wherein a halide of an element of group IIA, IIIA or IVA or IIB, IVB or VIII of the periodic table is used as Lewis acid.

5. A process as claimed in claim 4, wherein MgCl₂, MgBr₂, BF₃, AlCl₃, SnCl₄, ZnCl₂, ZnBr₂, TiCl₄, FeCl₃, CoCl₂ or CoBr₂ is used as Lewis acid.

6. A process as claimed in claim 2, wherein the reaction is carried out at from -30 to +30°C.

7. A process as claimed in claim 2, wherein the reaction is carried out in the presence of a ketone with 3 to 8 carbons, a nitrile with 2 to 7 carbons, an aliphatic or cycloaliphatic ether with 3 to 6 carbons or an ester of an aliphatic carboxylic acid with 1 to 4 carbons and an alcohol with 1 to 4 carbons as solvent.

8. The use of 2,5-bis(1,1-dialkoxy-2-propyl)-2,5-dihydrofurans of the formula I as claimed in claim 1 for preparing 2,7-dimethyl-2,4,6-octatrienedial.

9. The use of 2,5-bis(1,1-dialkoxy-2-propyl)-2,5-dihydrofurans of the formula I as claimed in claim 1 for preparing symmetric C₄₀-carotenoids of the formula IV 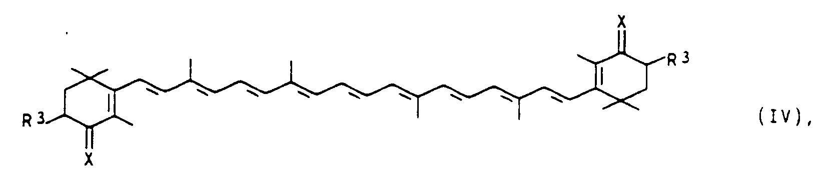 where R₃ is hydrogen or OH and X is two hydrogens or one oxygen.

10. A process for preparing symmetric C₄₀-carotenoids of the formula IV 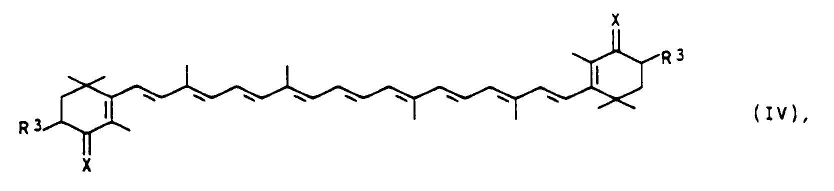 where R³ is hydrogen or OH, and X is two hydrogens or one oxygen, which comprises adding a C₁₅-triphenylphosphonium salt of the formula V 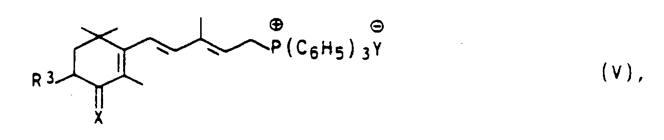 where R³ is hydrogen or OH, X is two hydrogens or one oxygen, and Y is an anion, to a 2,5-bis(1,1-dialkoxy-2-propyl)-2,5-dihydrofuran of the formula I 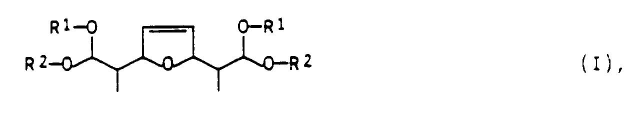 where R¹ and R² have the meanings stated in claim 1, and subjecting the dial formed thereby from the bisacetal of the formula I by hydrolysis to a Wittig reaction with the carbenium ion formed from the phosphonium salt of the formula V by addition of a suitable strong base, and, where appropriate, subsequently converting to the C₄₀-carotenoid by acid treatment.

## Revendications

1. 2,5-bis-(1',1'-dialcoxy-2'-propyl)-2,5-dihydrofurannes de formule générale I 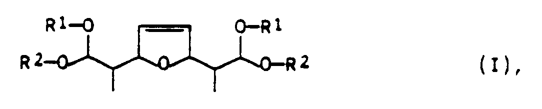 dans laquelle R¹ et R² peuvent être identiques ou différents et peuvent représenter chacun un groupe alkyle ayant de 1 à 4 atomes de carbone.

2. Procédé pour préparer des 2,5-bis-(1',1'-dialcoxy-2'-propyl)-2,5-dihydrofurannes de formule générale I selon la revendication 1, caractérisé en ce qu'on fait réagir un 2,5-dialcoxy-2,5-dihydrofuranne de formule générale II 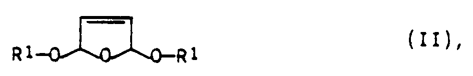 dans laquelle R¹ est un groupe alkyle ayant de 1 à 4 atomes de carbone, en présence d'un catalyseur acide et éventuellement d'un solvant, à une température comprise entre -78°C et le point d'ébullition du mélange réactionnel, avec un oxyde d'alkyle et de propényle de formule III 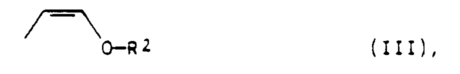 dans laquelle R² est un groupe alkyle ayant de 1 à 4 atomes de carbone.

3. Procédé selon la revendication 2, caractérisé en ce qu'on utilise comme catalyseur acide un acide protonique ou un acide de Lewis.

4. Procédé selon la revendication 3, caractérisé en ce qu'on utilise comme acide de Lewis un halogénure d'un élément du groupe IIa, IIIa ou IVa ou du groupe IIb, IVb ou VIII du Tableau Périodique des Eléments.

5. Procédé selon la revendication 4, caractérisé en ce qu'on utilise comme acide de Lewis MgCl₂, MgBr₂, BF₃, AlCl₃, SnCl₄, ZnCl₂, ZnBr₂, TiCl₄, FeCl₃, CoCl₂ ou CoBr₂.

6. Procédé selon la revendication 2, caractérisé en ce qu'on met en oeuvre la réaction à des températures de -30 à +30°C.

7. Procédé selon la revendication 2, caractérisé en ce qu'on met en oeuvre la réaction en présence d'une cétone ayant de 3 à 8 atomes de carbone, d'un nitrile ayant de 2 à 7 atomes de carbone, d'un éther aliphatique ou cycloaliphatique ayant de 3 à 6 atomes de carbone ou d'un ester d'un acide carboxylique aliphatique ayant de 1 à 4 atomes de carbone et d'un alcool ayant de 1 à 4 atomes de carbone, servant de solvant.

8. Utilisation des 2,5-bis-(1',1'-dialcoxy-2'-propyl)-2,5-dihydrofurannes de formule générale I selon la revendication 1, pour préparer le 2,7-diméthyl-octa-2,4,6-triène-1,8-dial.

9. Utilisation des 2,5-bis-(1',1'-dialcoxy-2'-propyl)-2,5-dihydrofurannes de formule générale 1 selon la revendication 1, pour préparer des caroténoïdes symétriques en C₄₀ de formule générale IV 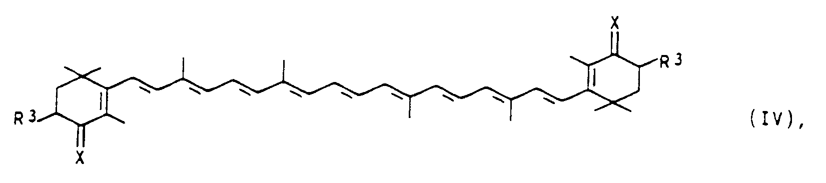 dans laquelle R³ est un atome d'hydrogène ou le groupe OH, et X représente deux atomes d'hydrogène ou un atome d'oxygène.

10. Procédé pour préparer des caroténoïdes symétriques en C₄₀ de formule générale IV 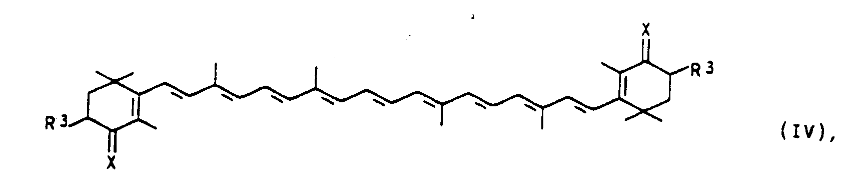 dans laquelle R³ est un atome d'hydrogène ou le groupe OH, et X représente deux atomes d'hydrogène ou un atome d'oxygène, caractérisé en ce qu'on ajoute d'abord à 2,5-bis-(1',1'-dialcoxy-2'-propyl)-2,5-dihydrofurannes de formule générale I 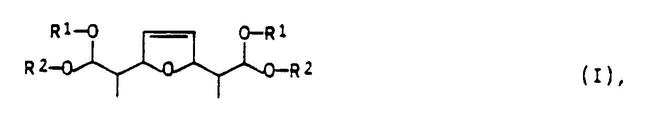 dans laquelle R¹ et R² ont les significations données dans la revendication 1, un sel de triphénylphosphonium en C₁₅ de formule générale V 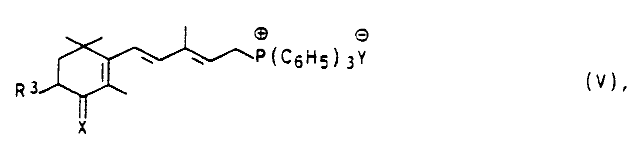 dans laquelle
R³ est un atome d'hydrogène ou un groupe OH,
X représente deux atomes d'hydrogène ou un atome d'oxygène, et
Y est un équivalent d'un résidu d'acide approprié
puis qu'on fait réagir le dial ainsi formé par hydrolyse à partir du bisacétal de formule I, par addition d'une base forte appropriée, dans le cadre d'une réaction de Wittig, avec l'ion carbénium formé à partir du sel de phosphonium de formule V, suivie éventuellement d'un traitement par un acide, pour obtenir le caroténoïde en C₄₀.
